# EUROPEAN PATENT APPLICATION

(11) **EP 4 643 869 A1**
(43) Date of publication of application: **05.11.2025**
(21) Application number: 24174080.2
(22) Date of filing: 03.05.2024
(51) Int. Cl.: A61K 38/09, A61K 31/513, A61K 47/00, A61P 13/00, A61P 13/08

(54) **USE OF GNRH ANTAGONISTS SUCH AS TEVERELIX TO TREAT ACUTE URINARY RETENTION**

(71) Applicant: Antev Limited, Weybridge KT13 8AH (GB)
(72) Inventor: MacLean, Carol, London, W1S 1DN (GB)
(74) Representative: Holme Patent A/S

(57) **Abstract**

The present invention relates to a composition comprising at least one GnRH antagonist for use in reducing the risk of recurrent acute urinary retention (AUR) in males of least 45 years of age presenting with a first episode of AUR. Said composition can within four week effectively alleviate urethral obstruction caused by an enlarged prostate, and restore normal urine flow without the need for surgery.

## Description

Acute retention of urine (AUR) is a urologic emergency characterised by a sudden inability to pass urine. AUR not only leads to severe pain/discomfort but also involves potential complications, such as loss of bladder contractibility, urinary tract infections, kidney damage, incontinence, urosepsis, increased risk of another episode of AUR, and psychological distress.

The incidence of AUR increases with age and its prevalence is significant, worldwide affecting approximately 1% to 6% of men over the age of 40 annually. A United Kingdom database study (Cathcart et al. Incidence of primary and recurrent acute urinary retention between 1998 *and* 2003 in England. J Urol. 2006;176(1):200-4; discussion 4), reported an annual incidence rate of primary acute urinary retention in men at 3.06 per 1,000, with 65.3% of cases being spontaneous. The most important finding in this study, which merits emphasis, is that men who have an episode of benign prostatic hyperplasia (BPH) related AUR are at significant risk for another episode within 6 months.

Furthermore, AUR patients have a mortality that is 4-5 times higher after 3 months and 2-3 times higher after 1 year of AUR compared with the general population. This is especially true in younger patients (Bengtsen et al. Long-term risk of benign prostatic hyperplasiarelated surgery and acute urinary retention in men treated with 5-alpha reductase inhibitor versus alphablocker monotherapy in routine clinical care. Prostate 2023; 83: 980-989). Clinically even one episode can result in permanent damage to the bladder with longstanding pathologic effects and symptoms (Choong S and Emberton M. Acute urinary retention. BJU Int. 2000; 85: 186-201).

When a patient experiences an episode of AUR, the bladder must immediately be allowed to empty by inserting a urinary catheter. The treatment is then followed by medical therapy and a subsequent TWOC (Trial Without Catheter), in order to see if further episodes of AUR can be prevented. However, this is often not the case, and the need for repeated catheterizations or medical interventions is burdensome and affects daily activities and social interactions. The unpredictable nature of AUR recurrence further adds to the distress and anxiety experienced by patients.

In addition to impacting patient's quality of life, the repeated catheterizations, emergency department visits, and hospital admissions necessary for patients experiencing multiple AUR episodes impose a substantial burden on healthcare systems.

The etiology of AUR is poorly understood, although it often arises as a result of underlying medical conditions such as enlarged prostate, urinary tract infections, or neurological disorders. Risk factors include age, elevated prostate specific antigen (PSA) and chronic inflammation. BPH, which can lead to bladder neck obstruction, is the most common cause, accounting for ~60% of AUR cases (Muruganandham et al. Acute urinary retention in benign prostatic hyperplasia: Risk factors and current management. Indian J Urol 2007; 23: 347-353).

The primary treatment goals for men with AUR are to decompress the urinary bladder, alleviate lower urinary tract symptoms and prevent the progression of the disease, in particular the need for surgery. The risk of progression is directly related to the prostate volume. Therefore, drugs that reduce prostate volume have been shown to exhibit the greatest effect in preventing the disease progression. Two classes of drugs, α-blockers and 5-α-reductase inhibitors, are presently approved by authorities for treatment of symptoms of BPH.

It is believed that α1 receptors influence lower urinary tract function not only through the direct effect on smooth muscle but also at the level of the spinal cord, ganglia, and nerve terminals to influence sympathetic, parasympathetic, and somatic outflows to the bladder, bladder neck, prostate, and external urethral sphincter.

However, studies have shown that α-blockers do not provide a benefit on AUR. For instance, the α1-blockers agent alfuzosin only showed a small effect on AUR in The ALFAURUS Phase 3 study, sponsored by Sanofi. Said study enrolled 806 subjects with a first episode of AUR related to BPH and was conducted between 2001 and 2004. It was designed to assess the efficacy of alfuzosin 10 mg once daily (OD) in the management of AUR associated with BPH. It was a double-blind, placebo controlled, randomized, multicenter study comparing two parallel groups (alfuzosin 10 mg OD or placebo) in patients with a first episode of AUR related to BPH. The acute episode was managed with catheterization and with study drug treatment for 2 to 3 days, followed by an active voiding trial to assess the patients' ability to void after catheter removal (i.e. TWOC [trial without catheter]). Those patients who successfully voided continued their randomized treatment for a total treatment duration of 6 months. However, the success rate after 6 months (successful TWOC and no AUR relapse nor any need for surgery) was 43.5% for the alfuzosin treated patients and 39.7% for the placebo treated patients (Xatral SR Product Information Australia. 2022). Thus, there was only a very small effect of the α1-blocker agent, and the trial failed to meet its efficacy target.

The 5-α-reductase inhibitors, which have been shown to reduce prostate volume and reduce the risks of acute urinary retention and need for BPH surgery, have a very slow reaction time, prolonging the time it will take before the patients obtain some kind of relief, i.e. before the prostate volume is reduced sufficiently in order to have an effect on AUR episodes. Due to this slow action of the 5-α-reductase inhibitors, 45-60% patients with a first episode of AUR are reported to have second episode of AUR within a year (Kaplan. Expert Forum Position Statement. Unmet Medical Need: Recurrence of AUR in BPH patients. 2023). 5-α-reductase inhibitors are accordingly ineffective in preventing recurrent AUR, especially in view of the severity further episodes of AUR have on a patient, e.g. higher mortality rate etc.

Currently, there are no treatments that are specifically aimed at preventing recurrence of AUR, irrespectively of the cause.

While catheterization provides temporary relief, it is not a long-term solution. Furthermore, the current therapies are ineffective in a fast and efficient manner reducing the risk of recurrent AUR, and since even one episode of AUR may result in permanent damage to the bladder it is essential to prevent repeated episodes of AUR.

Therefore, there is an urgent need to prevent recurrent AUR independent of long-term BPH management, and there is an increasing demand to provide compositions capable of preventing or reducing recurrence of AUR already after a single episode of AUR.

Accordingly, a first aspect of the present invention is to provide a composition for reducing the risk of recurrence of acute urinary retention (AUR), and wherein said composition has an improved safety profile and an improved patient compliance.

It is a second aspect of the present invention is to provide a composition for preventing recurrence of acute urinary retention where an enlarged prostate is the primary cause of the AUR.

It is a third aspect of the present invention to provide a more effective composition for preventing recurrence of acute urinary retention, that can alleviate urethral obstruction and restore normal urine flow without the need for surgery.

These and further aspects are obtained by providing a composition comprising at least one GnRH antagonist for use in reducing the risk of recurrent acute urinary retention (AUR) in males of least 45 years of age presenting with a first episode of AUR.

GnRH antagonists compete with the endogenous neurohormone GnRH (also known as luteinizing hormone releasing hormone, LHRH) for binding to its receptors in the anterior pituitary gland. By decreasing or blocking GnRH action, GnRH antagonists suppress release from the anterior pituitary gland of follicle stimulating hormone (FSH) and luteinizing hormone (LH). In males FSH plays an important role in spermatogenesis and LH stimulates production of testosterone in the testes.

Even though both antagonists and agonists may bind to the GnRH receptors in the pituitary gland, the antagonist will immediately block the receptor whereas the agonist operates by first stimulating the receptor, and then "exhausting" the receptor to stop hormone secretion. Thus, GnRH antagonists have an immediate onset of action leading to a fast and profound suppression of testosterone and are therefore especially valuable in a rapid reduction in prostate volume with the potential to improve outcomes for patients with an episode of AUR and enlarged prostate, and accordingly reducing the risk of recurrent acute urinary retention (AUR) without the need for surgery.

The at least one GnRH antagonist will provide a reduction in the patients testosterone level, subsequently reducing the volume of the prostate. However, since testosterone is also responsible for a number of male characteristics, reduced and/or low testosterone levels provides a number of physiological changes, such as diminished interest in sex, impotence, reduced lean body mass, decreased bone density, lowered mood, and energy levels. Accordingly, chronic treatment with GnRH antagonists is as such not viable in the relevant population, i.e. in males of least 45 years of age, as this would lead to symptoms of hypogonadism.

However, short-term decrease in testosterone levels after administration of GnRH antagonists is acceptable for the benefit of preventing the next AUR episode in this population, as this will minimise the side effects of low testosterone levels as much as possible. The inventors of the present invention has found that a single dose of the composition comprising a pharmaceutically effective amount of the at least one GnRH antagonist is sufficient to provide an optimised dose-response relationship in which relatively low doses of the at least one GnRH antagonist can be used to provide a rapid reduction in prostate volume, preferably within four weeks, and at the same time reduce the side effect (adverse events) conventionally associated with GnRH antagonists.

Within the context of the present invention the term "single dose" means that the composition according to the invention is intended for being administered only once, rather than multiple times over a specific period e.g. at regular intervals. The composition may of course be administered several times in case a subject (e.g. a male patient) later, e.g. several months or years later may benefit from an additional administration, but it is preferred that that a single dose is enough to provide the reduced prostate volume required to reduce the risk of recurrent AUR. It is furthermore preferred, that said composition is not administered at regular intervals.

Thus, in contrast to the treatments known in the art where the patients suffering from AUR have to receive daily or weekly administrations of the α-blockers or 5-α-reductase inhibitors, the composition according to the present invention only requires administration of a single dose in order to reduce the risk of recurrent acute urinary retention (AUR) in males over 45 years of age presenting with a first episode of AUR.

Within the context of the present invention the term "pharmaceutically effective amount" refers to an amount of the at least one GnRH antagonist, which is sufficient to provide a reduction in prostate volume, preferably within four weeks after administration of a single dose, and thereby reduce the risk of recurrent AUR.

The pharmaceutically effective amount will vary depending upon such factors as the potency of the particular GnRH antagonist or combination of the GnRH antagonists, the desired physiological or pharmacological effect, the route of administration and the time span of the intended treatment. Those skilled in the arts will be able to determine the pharmaceutically effective amount for any given GnRH antagonist(s) in dependence of these parameters, in accordance with standard procedures.

Within the context of the present invention the term "reduced risk of recurrent AUR" refers to a reduction in recurrent AUR episodes when compared to male patients having presented with an episode of AUR that does not receive any treatment. The term may alternatively, or in addition, also refer to a delay in a recurrent episode of AUR, e.g. if a recurrent episode is inevitable and the composition according to the invention is able to delay its occurrence and/or delay the need for surgery.

It is in this respect preferred that a single dose of the composition according to the invention comprises a pharmaceutically effective amount of the at least one GnRH antagonist sufficient for provide a reduction in the prostate volume of a male receiving said composition, and wherein the reduction in prostate volume is at least 10% after four weeks, when being compared to the prostate volume of said male before the composition was administered. In a preferred embodiment the prostate volume is reduced with at least 15% and even more preferred at least 20%.

The inventors of the present invention expect that at least 80% of all patients receiving the composition according to the present invention will obtain a clinical benefit of said composition. The inventors of the present invention further believes that a reduction in the prostate volume of at least 10% is will reduce the risk of having one or more recurrent AUR episode(s) with at least 30% compared to patients that does not receive any treatment. If the prostate volume is reduced e.g. about 15 % the risk of a further episode of AUR is reduced with about 50% and a reduction in the prostate volume of about 20 % ensures that the risk of a further episode of AUR is reduced with about 70%.

A person skilled in the art will understand that these numbers are mean of a large number of subjects where BPH is the primary reason for the AUR episode, and that variation in reduction in prostate volume and reduced risk of recurrent AUR will vary, among others in depends of the age and health of the subject.

The inventors of the present invention has found that although a reduction in the testosterone concentration is required in order to obtain the desired clinical efficacy, it is not needed to lower the testosterone to castrate level to achieve the optimal efficacy. It is accordingly preferred that that the pharmaceutically effective amount of the at least one GnRH antagonist does not clinical castrate a subject (or only clinically castrate a low number of subjects, e.g. below 30%) receiving the composition i.e. the subject will maintain a blood plasma testosterone concentration at or above 0.5 ng/mL, determined by a liquid chromatography/mass spectrometry (LC-MS) assay, after receiving the composition. Testosterone level ≤ 0.5 ng/ml has been used to define castrate level after surgery or after androgen deprivation treatment (ADT) in metastatic prostate cancer.

Furthermore, use of the composition according to the invention will ensure that the testosterone level will not remain reduced or low, but return to the baseline levels in less than eight weeks, thereby substantially preventing the side effects of low testosterone levels, and at the same time ensure that the risk of recurrent AUR is reduced, preferably prevented.

Thus, the composition is not only able to reduce the prostate volume within four weeks, thereby reducing (potentially preventing) recurrent AUR, but also prevent problems of long-term low testosterone levels in a male patient.

The at least one GnRH antagonist is preferably selected from the group comprising teverelix, abarelix, cetrorelix, degarelix, ganirelix, elagolix and relugolix, or a salt thereof. However, in a preferred embodiment according to the invention the GnRH antagonist is N-Ac-d-Nal¹,d-pCl-Phe²,d-Pal³,d-(Hci)⁶,Lys(iPr)⁸,d-Ala¹⁰ trifluoroacetate, i.e. teverelix-trifluoroacetate or teverelix-TFA.

The inventors of the present invention has shown that dosage regimes using two single injections of 60 mg teverelix 48 hours apart, administered subcutaneously are able to treat patients suffering from BPH and said inventors have shown that the prostate volume is decreased by 11.5% after administration.

This reduction occurred within four weeks of initiating therapy. By reducing the physical bulk of the prostate, the obstruction and urinary symptoms were reduced.

However, even though this dosage regime provided beneficial results it is preferred that the patients only require a single administration, i.e. a single dose of the composition according to the invention, i.e. the composition can be administered during a single visit at e.g. a medical facility, instead of the patient having to arrive at said medical facility two days apart in order to obtain sufficient serum concentrations for achieving the desired effect.

In this way, the composition according to the invention is unique since a single dose of said composition is sufficient for relieving the patient of the AUR symptoms, preferably after about four weeks, and at the same time preventing problems of long-term low testosterone levels in a male patient. For instance, teverelix-TFA will within four weeks reduce the risk of a recurrent episode of AUR and at the same time improve Lower Urinary Tract Symptoms (LUTS).

The single dose of the at least one GnRH antagonist, e.g. teverelix-TFA is preferably in the range of 60 to 180 mg, preferably in the range of 80 to 130 mg, such as e.g. 90 mg or 120 mg. Said dose is preferably administered subcutaneously (SC) or intramuscularly (IM). Said single dose is preferably placed in a suitable syringe in order to provide an easy administration.

Intramuscular administration provides a fast absorption rate compared to the slower release peak of a subcutaneously administration. Even though the differences between PK profiles following subcutaneous or intramuscular dose scenarios become normalized over a period, the inventors have found that the PK profile in the first four weeks is important for providing a rapid reduction in prostate volume, thereby reducing the risk of recurrent AUR.

The inventors have in this respect found that an intramuscular administration of teverelix-TFA, e.g. an injection in the buttock, will provide high plasma levels of teverelix during the first four weeks and low/no plasma levels of teverelix during the following weeks. Thus, intramuscular administration will provide a fast response. In comparison a subcutaneous administration e.g. injection in the lower abdominal wall, will provide lower plasma levels of teverelix during the first four weeks (compared to the intramuscular injection) but a sustained low plasma level of teverelix during the following weeks, thereby providing a sustained response.

Furthermore, bioavailability of teverelix following intramuscular injection is likely higher than bioavailability of teverelix following subcutaneous injection.

Thus, the physician may select the most optimal administration route in depending on the need of the subject, e.g. the fast action of an intramuscular administration or the sustained action of the subcutaneous administration. However, even though it is preferred that the subject only receives a single administration (injection) either subcutaneous or intramuscular, a subject may in some situations benefit from a dual administration i.e. both a subcutaneous and intramuscular administration, in order to obtain a longer effect of the composition.

In one embodiment, when the composition according to the invention is administered subcutaneous, it is preferred that said composition comprises a higher concentration of the at least one GnRH antagonist compared to a composition which is administered intramuscular.

In a preferred embodiment the composition comprises 120 mg teverelix-TFA when administered subcutaneous and 90 mg teverelix-TFA when administered intramuscular.

The inventors of the present invention has demonstrated that the PKPD profile of a single dose of 120 mg teverelix-TFA administered subcutaneous is comparable to the PKPD profile observed with two subcutaneous injections of 60 mg teverelix-TFA administered two days apart.

Furthermore, the inventors have observed that a single 90 mg intramuscular injection of teverelix-TFA provided higher plasma levels of teverelix during the four weeks following injection and the majority of trial subjects had low testosterone levels below 0.5 ng/mL for around four weeks. After four weeks plasma levels of teverelix declined rapidly and testosterone levels recovered quickly. Thus, delivering e.g. teverelix via the intramuscular route of administration affords the potential for a greater reduction in prostate volume due to initial higher plasma exposure following intramuscular injection compared with subcutaneous injection

Without being bound by theory, it may be that the reduction in prostate volume (size) is a result of an increase in apoptotic index and higher initial levels of teverelix may accordingly elicit a greater apoptotic effect and an increased reduction in prostate volume.

It has been found that the plasma concentration of teverelix in different patients is substantially independent of the patient's weight, and it is accordingly believed that a unit dose (same dose) of teverelix-TFA is considered to be universal for all subjects.

For the at least one GnRH antagonist, e.g. teverelix-TFA, to be absorbed in the body, said GnRH antagonist(s) must preferably be present in the form of solution at the site of absorption. Various techniques are used for the enhancement of the solubility of poorly soluble drugs, such as GnRH antagonists.

In relation to teverelix-TFA, it has been found that crystal engineering, in which the teverelix and trifluoroacetate (TFA) provides an organisation of the teverelix peptide and its counter-ion TFA in a crystalline structure provides an effective sustained release of teverelix, having improve physicochemical properties (e.g., solubility and stability), and improve efficacy (e.g., bioavailability).

However, teverelix has a tendency of forming gels in the presence of counter ions. Thus, in order to obtain the desired microcrystalline suspension without any undesirable gel-formation, the applicant has proven see e.g. WO2020007852 and WO2020007857 that when the molar ratio of teverelix to the counter-ion trifluoroacetate (TFA) is at least 1:2, preferably at least 1:2.2 and preferably not above 1:2.8 a stable, homogenous microcrystalline suspension is provided that does not contain any gel. Furthermore such suspensions will comprise both soluble and insoluble teverelix, thereby providing a unique bioavailability of teverelix.

Different methods to obtain and adjust said molar ratios to the desired molar ratios according to the invention, are disclosed in the applicant's applications WO2020007852 and WO2020007860.

Within the content of the present invention the term "molar ratio of teverelix to trifluoroacetate" refers to the molar relationship between teverelix and trifluoroacetate, where the first number of the molar ratio is the mol content of teverelix in the composition and the second number refers to the mol content of TFA in the composition. For instance, a molar ratio of 1:2.2 means that for each mol teverelix in the composition, said composition comprises 2.2 mol TFA, and a molar ratio of at least 1:2.2 means that for each mole teverelix in the composition, the composition comprises at least 2.2 mol trifluoroacetate (TFA).

Without being bound by theory, the soluble teverelix is in the form of an aqueous solution, and in some situations a gel. The presence of a gel will inhibit any freely aqueous teverelix and therefore prevent, or at least reduce, immediate release. The insoluble teverelix is in the form of microcrystals. Said microcrystals will prevent gel formation, therefore "unlocking" the aqueous teverelix. Over time the TFA in the composition according to the invention will be absorbed by the body, lowering the ratio, so the microcrystals subsequently turn in to gel, which forms the slow release depot. Thus, the non-gel-soluble teverelix is immediately available, providing an almost immediate onset of action, and the gel-soluble and insoluble teverelix (microcrystals) will assist in providing a sustained release of teverelix. Accordingly the composition according to the invention provides a soluble-insoluble transition at the administration site, and accordingly a sustained release of teverelix.

Thus, when teverelix-TFA is the GnRH antagonist in the composition according to the present invention, said composition has both an immediate onset of action leading to a fast suppression of the gonadotropins, but also a sustain release of teverelix thereby ensuring that the subject maintains a therapeutically effective concentration in the blood plasma to provide the desired reduction in prostate size. This will not only provide a more reliable composition for the reducing the risk of recurrent AUR, but also improve patient compliances as only a single administration (e.g. injection) is required. It is in this respect preferred that the patient can be treated in a single visit, i.e. that said patient does not have to visit a medicinal facilities several consecutive days. Even though the patient may be any kind of primate, it is preferred that the patient is a male human. AUR is very rare in women, but the composition according to the present invention can also be used to reduce the risk of recurrent AUR in women.

The composition will in addition to reducing the blood plasma testosterone concentration preferably also reduce the subject's blood plasma prostate specific antigen (PSA) concentration to less than about 4 ng/mL. PSA is a substance made by cells in the prostate gland. PSA is mostly found in semen, but a small amount is also found in the blood. Incidence of an enlargement of a prostate, such as through a condition such as BPH, can increase an amount of PSA in a subject. Therefore, measuring PSA levels in a subject is a way to monitor how patients with enlarge prostate are responding to treatment.

The present invention also relates to a pharmaceutical composition comprising the composition according to the invention, and in which the pharmaceutical composition further comprises a pharmaceutically acceptable excipient, diluent, or carrier.

The diluent may in a simple embodiment be water but can also be an aqueous buffering agent, e.g. saline, citrate, phosphate, acetate, glycine, tris(hydroxymethyl)aminomethane (tris), 4-(2-hydroxyethyl)-1-piperazineethanesulfonic acid (HEPES) and/or piperazine-N,N'-bis(2-ethanesulfonic acid) (PIPES).

In some embodiments, the formulation further comprises an isotonic agent, e.g. a polyhedric alcohol, a sugar alcohol and/or mannitol.

In some aspects, the composition or formulation can be packaged in a container e.g. a vial, and can further comprise at least one syringe and needle for administration of said composition or formulation. In some exemplary embodiments, a pair of needles can be provided of proper length for injection via intramuscular or subcutaneous injection.

In some embodiments, the composition or formulation can be provided as a lyophilized powder contained in a vial, e.g. obtained via the method described in WO2020007860. A kit can comprise a vial of sterile water or TFA-solution for reconstitution/resuspension of the lyophilized composition, as well as instructions for preparation. In other instances, a syringe comprising an integrated water chamber can be provided to re-suspend the lyophilized composition or formulation within the syringe prior to administration.

A better understanding of present invention will be obtained by reference to the following discussion of the provided experimental results.

### Experiments:

Teverelix-TFA has been studied in 450 male and female subjects in 16 Phase 1 and Phase 2 clinical trials. Teverelix-TFA has been tested in various dosing regimens in Phase 2 clinical trials in 97 patients with BPH. Across a total of 1,117 injections administered via a subcutaneous or intramuscular route, teverelix-TFA has been found to be safe and well-tolerated with no Suspected Unexpected Serious Adverse Reactions (SUSARs) being reported to date.

Administrations have ranged from a single dose of 0.125 mg up to 180 mg repeat doses of teverelix for three consecutive days. Phase 1 and 2 pharmacodynamic efficacy data generated in adult males and females demonstrate the ability of teverelix to modulate sex steroid hormones in a dose-dependent manner.

### Experiment I

This was a multicentre (n=8), randomised, double-blind, placebo-controlled study in treatment of patients 50 years or over, with BPH and bothersome voiding symptoms characterized as an International Prostate Symptom Score (IPSS score ≥13) and a uroflow <13 mL/sec for a voided volume ≥150 mL. Exclusion criteria included an urgent need for prostate surgery, elevated prostate specific antigen (PSA) levels above the upper limit of normal relative to age, major organ dysfunction, prior surgical treatment of the prostate or bladder, and a large residual urine volume (>350 mL).

The study comprised two periods: a four-week single-blind placebo run-in period for all patients to remove any placebo responders and reduce between-patient variability in baseline measurements. This was followed by a 16-week randomised, double-blind, placebo-controlled, parallel group period for eligible patients. Following screening (Day-28), patients entered the placebo run-in period and received two subcutaneous injections of placebo, 48 h apart. During this period, one visit was planned for training purposes (e.g., IPSS). At the end of the placebo run-in period, patients still meeting the eligibility criteria were randomised to receive two subcutaneous injections of 60 mg teverelix or placebo, 48 h apart, on day 1 and day 3. Teverelix was supplied as a freeze-dried powder that was reconstituted with 5% mannitol in water for injection. Corresponding placebo formulations, without teverelix-TFA, were also prepared. Injections were given in the abdomen. Patients attended follow-up visits for up to 16 weeks.

### ASSESSMENTS

Baseline values of all parameters were obtained at the end of the placebo run in period (Day 1).

The IPSS was assessed at various time points, including screening (Day-28), during the placebo run-in (Day-14), and at Weeks 1, 2, 4, 8, 12, and 16. The IPSS questionnaire comprised seven questions about urinary symptoms such as incomplete emptying, frequency, urgency, weak stream, and nocturia. Each symptom was rated from 0 to 5 based on severity. Symptoms are categorized as mild (≤7), moderate (8-19) or severe (20-35). (15)

Urinary flow rate (Qmax) was also measured at corresponding time points using uroflow meters.

Prostatic volume was evaluated at Day 1, Week 4, and Week 16 via transrectal ultrasound.

QoL was assessed using the last IPSS question that refers to the patient's perceived QoL by asking the following: "If you were to spend the rest of your life with the urinary conditions just the way it is now, how would you feel about that?" Responses were scored from 0-6 (0=delighted, 1=pleased, 2=mostly satisfied, 3=mixed, 4=mostly dissatisfied, 5=unhappy, 6=terrible).

At the final assessment, the investigator assessed efficacy and tolerability as either very good/good, satisfactory, poor/insufficient, or not assessable.

Blood samples were gathered in the morning on Day 1 and Day 3 prior to dosing, along with assessments at the conclusion of Weeks 1, 2, 3, 4, 8, 12, and the final evaluation at Week 16, for the measurement of teverelix, testosterone, and dihydrotestosterone (DHT). Plasma concentrations of teverelix were analysed to delineate the release profile of the active substance from teverelix.

Safety included the assessment of adverse events (AEs), vital signs (supine blood pressure and pulse), electrocardiograms (ECG), and clinical laboratory parameters (haematology, clinical chemistry, and urinalysis). Drug-related AEs were defined as those with a relationship to study drug of `likely' or `not assessable'.

### STATISTICAL ANALYSIS

### Sample size

It was assumed that due to the run-in phase the placebo effect could be reduced to a mean improvement of 3 IPSS points in the second part of the study. Based on this, a difference in the mean IPSS of 4 to 5 between the teverelix group and the placebo group, a corresponding common SD of 6 to 7, and a effect size of 0.667 to 0.714, the needed sample size was estimated to be at least 35 to obtain a 80% statistical power for a 2 sided -t test with α set at 0.05.

### Data set and statistical tests

The primary analysis was performed for the intent-to-treat dataset. The robustness in results was supported by the per-protocol analysis and therefore these results are not reported here.

For all parameters summary statistics were calculated (e.g. mean, SD, median, minimum, and maximum). Post-hoc, a clinically relevant reduction in the IPSS was defined as an IPSS score reduction of three or more points and the percentage of patients with a clinically relevant reduction was calculated. The change in the IPSS at Weeks 2, 4, 8, 12, and the final assessment compared with baseline; the percentage change in the IPSS from baseline; the change in maximum and mean urinary flow rate at Weeks 2, 4, 8, 12, and the final assessment; and the change in total prostatic volume at Week 4 and the final assessment was analysed by analysis of covariance with treatment group, centre as factors, and baseline values as cofactors. QoL scores at Weeks 2, 4, 8, 12, and the final assessment were analysed using a logit model with treatment group, centre, and treatment group by centre interaction as terms. All predefines statistical analyses were conducted using SAS^{®} software (v8.2) (SAS Institute Inc., Cary, NC, USA). The percentage IPSS responders between groups were compared using a chi-square test for proportions (https://www.medcalc.org).

The treatment group by centre interaction was not statistically significant at almost all time points, and therefore the results are presented for all centres combined. Exceptions were the treatment group by centre interaction for the IPSS and the percentage change in the IPSS, which were found to be statistically significant at the 10% significance level at Week 2 and Week 4, and at Week 2, respectively.

### RESULTS

### Patient population

A total of 101 patients were screened and entered the placebo run-in period. Of them, a total of 81 with a mean age of 67.4 years were randomised and entered the treatment period, 41 patients in the teverelix group and 40 patients in the placebo group. Seventy-nine patients completed the study. One patient was withdrawn as a result of intercurrent disease, and one patient was withdrawn for other reasons not related to the treatment, both in the placebo group. Treatment groups were similar at baseline with regard to age, symptom score, prostatic volume, PSA levels, and urinary flow rate. All subjects had moderate or severe BPH at study entry, see Table 1.

**Table 1. Baseline patient population characteristics**

| | Teverelix (n=41) | Placebo (n=40) |
|---|---|---|
| Age, years (SD) | 67.9 (5.3) | 67.0 (5.5) |
| IPSS (SD) | 18.4 (4.6) | 17.6 (4.1) |
| Prostate Volume, cm³ (SD) | 54.04 (23.99) | 48.35 (22.68) |
| PSA, ng/mL (SD) | 3.00 (2.50) | 2.98 (2.68) |
| Qmax, mL/sec (SD) | 4.96 (1.24) | 5.51 (1.39) |

| | | |
|---|---|---|
| IPSS, International Prostate Symptom Score; PSA, prostate-specific antigen; Qmax, maximum urine flow | | |

The teverelix-TFA formulation was reconstituted as follows. 0.8 ml 5% mannitol was added to 60 mg of the GnRH antagonist teverelix-TFA. The mixture was stirred using vortex during one minute providing a dose formulation of teverelix-TFA as a flowing milky pearly microcrystalline aqueous suspension. The suspension is made of microcrystals of about 10 um length. Corresponding placebo formulations, without teverelix-TFA, were also prepared

### Formulation A: 60 mg teverelix-TFA in 0.8 ml 5% mannitol Formulation A-placebo: 0.8 ml 5% mannitol

Two single injections of Formulation A and Formulation A-placebo was administered subcutaneous at an interval of 48 hours to 81 male patients (41 on A and 40 on A-placebo) suffering from BPH.

### EFFICACY PARAMETERS

### International Prostate Symptom Score

In the teverelix group, there was a consistent improvement in the IPSS compared to baseline throughout the study, see Table 2 below.

**Table 2. Mean and adjusted mean change from baseline in the International Prostate Symptom Score**

| | | Teverelix (N=41) | Placebo (N=40) | Adjusted treatment difference (95% CI) | p-value² |
|---|---|---|---|---|---|
| Week 2 | Mean (SD), n | -2.4 (3.2), 40 | -0.8 (1.9), 41 | -1.6 | |
| | Adjusted mean¹ | -2.1 | -0.5 | -1.6 (-2.6; -0.5) | 0.004 |
| Week 4 | Mean (SD), n | -4.5 (3.7), 41 | -0.9 (2.6), 39 | -3.6 | |
| | Adjusted mean | -4.4 | -1.1 | -3.3 (-4.6; -2.0) | <0.001 |
| Week 8 | Mean (SD), n | -5.4 (4.2), 41 | -1.2 (2.6), 39 | -4.2 | |
| | Adjusted mean | -5.2 | -1.4 | -3.9 (-5.2; -2.5) | <0.001 |
| Week 12 | Mean (SD), n | -5.7 (4.5), 41 | -1.4 (3.7), 37 | -4.3 | |
| | Adjusted mean | -5.6 | -1.6 | -3.9 (-5.5; -2.4) | <0.001 |
| Week 16 (final assessment) | Mean (SD), n | -6.3 (3.9), 41 | -1.1 (2.9), 38 | -5.2 | |
| | Adjusted mean | -6.3 | -1.4 | -4.9 (-6.1; -3.6) | <0.001 |

| | | | | | |
|---|---|---|---|---|---|
| ¹Represents adjusted mean from the ANCOVA model including treatment group and centre as factors and baseline I-PSS as cofactor ²Significance levels for the mean adjusted treatment difference | | | | | |

At the final assessment (Week 16), patients treated with teverelix experienced a notable mean score reduction of 6.3 (SD: ±3.9). In the placebo group the mean reduction was 1.1 (±2.9). The adjusted mean difference in the IPSS at the final assessment and all other time points was significant (p< 0.05 at Week 2 and p<0.001 at all other weeks).

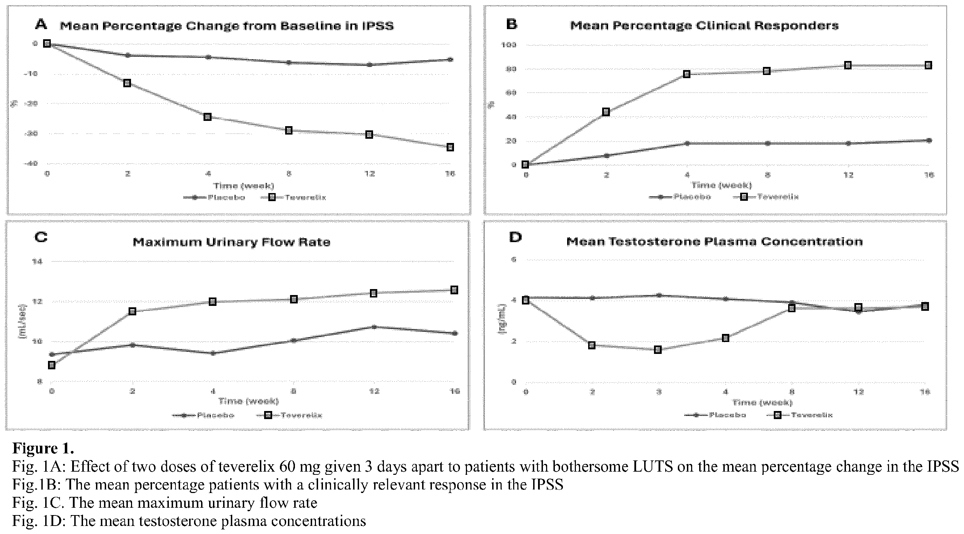

Moreover, at Week 2, the mean percentage change in the IPSS was 13.0% (±17.4%) in the teverelix group and 3.8% (±9.3%) in the placebo group. At the final assessment, this increased to 34.5% (±20.0%) and 5.2% (±15.9%), respectively, see Figure 1a. The adjusted mean difference in the percentage change in the IPSS at the final assessment and all other time points was significant (p<0.001).

Additionally, a significant clinically relevant reduction in the IPSS was evident in 43.9% of teverelix subjects by Week 2, this proportion increased to 75.6% by Week 4, and 82.9% at Week 16 (Figure 1b, p<0.001 at all time points).

### Flow rate

Throughout the study, patients in the teverelix group demonstrated at least a three times greater increase in Qmax rate compared to those in the placebo group (Figure 1c). At the final assessment Qmax was 3.7 mL/sec (±3.9 mL/sec) and 1.0 mL/sec (±3.1 mL/sec), in the teverelix and placebo group, respectively. The adjusted mean treatment difference at the final assessment and all other assessment points was significant (p<0.05).

Similarly, in terms of the absolute change in mean urinary flow rate, patients receiving teverelix experienced a greater improvement compared to those receiving placebo at all time points (data not shown). At the final assessment, Qmax was 2.0 mL/sec (±2.7 mL/sec) and 0.3 mL/sec (±2.0 mL/sec), in the teverelix and placebo group, respectively. The adjusted mean treatment difference at the final assessment and all other assessment points was significant (p<0.05)

### Prostatic Volume

Patients in the teverelix group exhibited a greater absolute change in total prostatic volume compared to the placebo group at Week 4 (teverelix: mean: -5.88 cm³ ; SD: ±7.7; placebo: mean: -2.52 cm³ ; SD: ±7.8). At the final assessment, the mean total prostatic volume decreased with 5.7 cm³ (±9.4 cm³) and 0.23 cm³ (±9.2 cm³) in the teverelix and placebo group, respectively. The adjusted mean treatment difference was significant at Week 4 (p<0.05) and the final assessment (p<0.001).

### Quality of life score

Overall, patients in the teverelix group reported improved QoL scores compared to those in the placebo group across all time points. The associated odds ratio was statistically significant at Week 4 (3.76, 95% CI: 1.53; 9.27, p< 0.01), Week 8 (6.83, 95% CI: 2.58; 18.11, p< 0.001), Week 12 (4.4, 95% CI: 1.76; 10.98, p< 0.001), and Week 16 (8.11, 95% CI: 3.11; 21.18, p< 0.001).

### Investigator rated the efficacy

Most of the patients in the teverelix group (73.2%) were rated as having very good/good efficacy by the investigator. In contrast, only 5% of the patients in the placebo group received this rating. Regarding tolerability, the investigator rated all patients in the teverelix group of patients as having very good/good tolerability, while in the placebo group (92.5%) received this rating.

### Pharmacokinetics and Pharmacodynamics

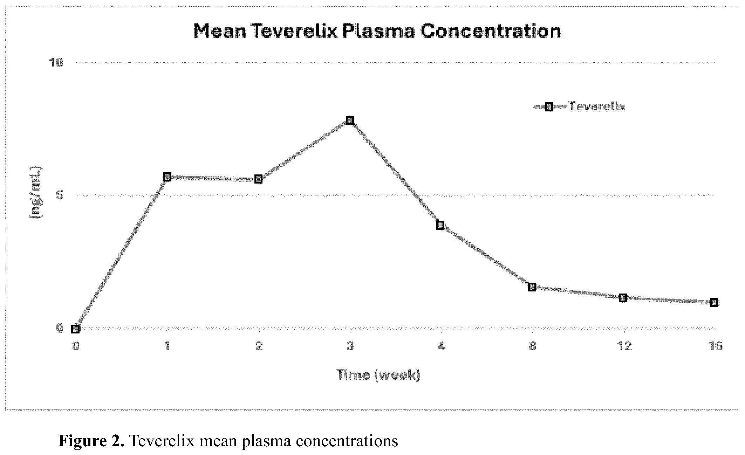

Following the initial administration of 60 mg teverelix, concentrations rapidly rose until Day 3 when the second dose was administered. This upward trend continued until the end of Week 3. Meanwhile, mean testosterone concentrations decreased, reaching 58.8% of baseline by Week 3, with the decline more pronounced than the increase in teverelix concentrations, see Figure 2. Subsequently, from the end of Week 3 onwards, teverelix concentrations decreased, coinciding with a rise in testosterone concentrations up to the final assessment at week 16, approaching baseline levels by week 8, see Figure 1d.

Mean PSA concentrations exhibited a slight overall decrease at the final assessment compared to baseline (teverelix: -0.27 (±1.46) ng/mL; placebo: -0.10 (±1.17) ng/mL).

### Safety

A total of 22 patients (53.7%) in the teverelix group reported an AE that was likely drug-related or not assessable. Most were subcutaneous injection site reactions (31.7%), pain (2.4%), and injection site induration (17.1%). Two drug-related AEs were reported in the teverelix group, one patient reported hyperuricaemia and 1 patient urinary retention. In the placebo group no subcutaneous injection site reactions were reported, 1 patient reported haematospermia which was considered drug related. All of the drug-related AEs were of mild intensity.

Four serious AEs were reported and assessed as not drug related: pyelonephritis, acute urinary retention, both in the placebo group and cholelithiasis/pancreatitis in the teverelix group.

There were no clinically significant changes in mean systolic or diastolic blood pressure or pulse during the study. None of the patient reported an AE related to vital signs. There were no notable changes in mean body weight or any of the ECG parameters (RR, QT, and QTc interval).

### DISCUSSION

The outcomes from this phase 2 trial indicate that teverelix provides a promising efficacious, well-tolerated, and relatively prompt intervention for addressing bothersome voiding symptoms in males aged over 45 with BPH. The treatment led to prostate volume reduction of 11%, almost full and persistent symptom relief, and exhibited only transient suppression of testosterone without any safety concerns.

In summary, teverelix-TFA competitively binds with endogenous GnRH at hypothalamic receptors results in a dose-dependent reduction in testosterone secretion and subsequently a reduction in the volume of the prostate.

In particular the following was shown:
- Rapid onset of effect (within 2 weeks).
- 11% reduction in prostate volume in 4 weeks.
- 40% improvement in uroflow.
- 4 weeks after first administration ~44% of teverelix subjects reported a clinically relevant (>3 point) reduction in International Prostate Symptom Score (I-PSS) compared with ~8% of placebo subjects. This rose to 76% (17.5% placebo) by Week 8 and 83% (17.5% placebo) by Week 12 and was maintained at Week 16 (83%/20%).
- Less than 29% of subjects had testosterone suppressed to castrate levels up to 4 weeks.

Therefore, the use of the composition according to the present invention is clearly successful in reducing prostate volume in a short period (<4 weeks), therefore ensuring that the risk of recurrent AUR is reduced.

The inventors of the present invention have demonstrated that the PKPD profile of a single dose of 120 mg teverelix-TFA administered subcutaneous is comparable to the PKPD profile observed with two subcutaneous injections of 60 mg teverelix-TFA administered two days apart, thus the results are also expected for a single dose of 120 mg teverelix-TFA administered subcutaneous, and also for single dose of 90 mg teverelix-TFA administered intramuscular.

One of the main advantages of the immediate onset of testosterone suppression is the lack of initial flare-up which is thought to be responsible for complications in some cases which did not occur in the present study.

An efficacious treatment will also take into account patient values and preferences and hence enhance their QoL. In this study, patients in the teverelix group already at Week 2 were more than twice as likely to give a favourable response compared to the placebo group. By the final assessment, patients in the teverelix group were more than 8 times as likely to provide a favourable response compared to patients in the placebo group.

Based on the results of this study and the pharmacological profile of teverelix the inventors find that teverelix-TFA may play a role in the management and prevention of BPH-related spontaneous AUR. Why a transient and short suppression of testosterone provides enduring effect of 6 months on prostate volume and concomitantly on the IPSS score and Qmax is not fully clear though. GnRH receptors have been identified in non-pituitary tissues, such as the prostate, potentially influenced by GnRH and its analogues employed in therapy.

There is a suggestion that their application results in reduced cell viability in the prostate, likely due to diminished proliferation and enhanced apoptosis, potentially mediated through mechanisms related to the cell cycle (Sakai et al. In search of the molecular mechanisms mediating the inhibitory effect of the GnRH antagonist degarelix on human prostate cell growth. PLoS One. 2015;10(3):e0120670).

According to another article (Colli E, Tank6 L. Gonadotropin-Releasing Hormone Antagonists: From Basic Science to the Clinic in Patients With Benign Prostatic Hyperplasia and Lower Urinary Tract Symptoms. UroToday International Journal. 2010;3 (5 (October))) other mechanisms of action include the lowering of estradiol levels with consequent antiproliferative action on the stroma the relaxation of the detrusor and prostatic smooth muscles.

The formulations used in the present invention are inexpensive to manufacture, and due to the ease of use it provides a very simple dosage regime.

Modifications and combinations of the above principles and combinations are foreseen within the scope of the present invention.

## Claims

1. A composition comprises at least one GnRH antagonist for use in reducing the risk of recurrent acute urinary retention in males over 45 years of age after presenting with a first episode of acute urinary retention.

2. A composition for use according to claim 1, wherein said composition is administered as a single dose.

3. A composition for use according to claim 2, wherein said composition is not administered multiple times over a specific period e.g. at regular intervals.

4. A composition for use according to any of the preceding claims, wherein a single dose of said composition comprises a pharmaceutically effective amount of the at least one GnRH antagonist sufficient for providing a reduction in the prostate volume of a male receiving said composition.

5. A composition for use according to claim 4, wherein the reduction in prostate volume is at least 10% after four weeks, when being compared to the prostate volume of said male before the composition was administered.

6. A composition for use according to any of the preceding claims, wherein a male receiving said composition maintains a blood plasma testosterone concentration above 0.5 ng/mL, determined by a liquid chromatography/mass spectrometry (LC-MS) assay.

7. A composition for use according to any of the preceding claims, wherein the blood plasma testosterone concentration returns to baseline after about eight weeks.

8. A composition for use according to any of the preceding claims, wherein the at least one GnRH antagonist is selected from the group comprising teverelix, abarelix, cetrorelix, degarelix, ganirelix, elagolix, relugolix, or a salt thereof.

9. A composition for use according to any of the preceding claims, wherein the at least one GnRH antagonist is teverelix-trifluoroacetate.

10. A composition for use according to claim 9, wherein the teverelix-trifluoroacetate composition is a microcrystalline suspension.

11. A composition for use according to claim 10, wherein said microcrystalline suspension comprises from 2.0 mol to 2.8 mol trifluoroacetate for each mol teverelix in said composition.

12. A composition for use according to any of the claims 9, 10 or 11 wherein the composition comprises 120 mg teverelix-trifluoroacetate when administered subcutaneous.

13. A composition for use according to any of the claims 9, 10 or 11, wherein the composition comprises 90 mg teverelix-trifluoroacetate when administered intramuscular.

14. A composition for use according to any of the preceding claims, where an enlarged prostate is the primary cause of the acute urinary retention, and/or wherein the use is independent of long-term benign prostatic hyperplasia management.

15. A pharmaceutical formulation comprising the composition according to any of the claims 1 - 14 for use in reducing the risk of recurrent acute urinary retention in males over 45 years of age after presenting with a first episode of acute urinary retention.
